# EUROPEAN PATENT SPECIFICATION

(11) **EP 4 587 089 B1**
(45) Date of publication and mention of the grant of the patent: **16.09.2026**
(21) Application number: 23801824.6
(22) Date of filing: 03.10.2023
(51) Int. Cl.: A61M 16/12, A61M 16/08

(54) **MEDICAL VENTILATOR**
MEDIZINISCHES BEATMUNGSGERÄT
RESPIRATEUR MÉDICAL

(30) Priority: 31.10.2022 GB 202216125
(43) Date of publication of application: 23.07.2025
(73) Proprietor: Oxford University Innovation Limited, Oxford, OX2 0JB (GB)
(72) Inventor: FARMERY, Andrew, Oxford OX3 9DU (GB); PITA, Alfonso Arturo Castrejon, Oxford OX1 3PJ (GB)
(74) Representative: Williams Powell
(86) International application number: PCT/GB2023/052550
(87) International publication number: WO 2024/094958

(56) References cited:
- WO-A1-2010/022363
- WO-A1-2015/128716
- WO-A1-2015/132682
- DE-A1- 102011 010 581
- DE-U1- 202022 100 332
- IT-A1- 202000 007 660
- US-A1- 2007 056 587
- US-A1- 2019 255 273
- US-B1- 10 314 999

## Description

The present application relates to a system for ventilating a user, and in particular to a pressure-controlled ventilator which does not employ any valves (such as breath actuated inspiration or expiration valves)

A medical ventilator is an apparatus for assisting with a patient's breathing by conveying air (or a mix of oxygen and other gases) into and out of the patient's lungs. If the air is pressurised then valve(s) are generally used to control the pressure, volume and flow of air to the patient.

The simplest ventilators include the so-called "bag in bottle" design which offer volume-controlled ventilation (VCV); in other words they deliver a fixed volume of air to the patient which is determined by the size of the "bag". They are therefore potentially adversely affected by any leaks in the system. Also, the bag has inertia, so changes in flow cannot be achieved easily, so it is best suited to constant flow delivery. VCVs can allow user-set characteristics such as tidal volume, rate, I:E ratio (inspiration/expiration), FIO2 (fraction of inspired oxygen) and PEEP (positive end-expiratory pressure: the pressure in the lungs above atmospheric pressure that exists at the end of expiration). Delivered volumes and pressure are monitored and this data informs the closed loop feedback controller, which adjusts outputs to match set-points.

Other historic VCV designs include those used in anaesthetics such as the Bains breathing circuit and the "Nuffield 200" produced by Penlon.

An alternative ventilation model is pressure-controlled ventilation in which the pressure of air delivered to the patient is controlled (a predetermined pressure is not exceeded). There are technical reasons why PCV may be easier to implement and control. Principally, this is because the sensor input informing the closed-loop controller is pressure, which can be measured easily and which has few calibration problems, as opposed to flow which is measured with less confidence. Secondly, in spontaneous mode, it is easier technically, and more acceptable in terms of patient tolerance, to offer pressure support in response to patient triggering than to offer a fixed tidal volume.

In standard ICU ventilators, flow is delivered by precision mass-flow controllers which can deliver flow rapidly to allow the pressure to be fixed at any value. There is an almost infinite supply of O2/air, so leaks are not an issue, but these systems are extremely expensive and uneconomical/wasteful in terms of oxygen use.

Valves are expensive, flimsy and sources of failure in ventilators and removing them would reduce the cost, speed up production and reduce the chance of ventilator failure.

WO 2015/132682 A1 (KONINKL PHILIPS NV) discloses a system configured to amplify the pressure and/or flow rate of a pressurized flow of breathable gas by entraining oxygen gas and/or ambient air with an air amplifier and a venturi valve at or near a blending gas source, distally (e.g., remotely) from an interface appliance of a subject interface (e.g., away from the face of the subject) to reduce noise from the system heard by the subject.

Further ventilatory support devices employing a Venturi mixer of the prior art are disclosed for example in US2007/056587 A1, WO2015/128716 A1, US2019/255273 A1 and WO2010/022363 A1.

### Summary of invention

The invention is defined in the appended independent claim 1. Preferred embodiments are matter of the dependent claims.

In accordance with a first aspect of the invention, there is provided a system for ventilating a user, including a port for supplying a mixture of gases to the user for inspiration and for receiving a mixture of gases from the user after expiration a first tube having an inlet for receiving at least one gas to ventilate the user and an outlet in fluid communication with said port for supplying said at least one gas to the port for inspiration by the user, a second tube having a first end proximate to and in fluid communication with said port and a second end distal to said port, a venturi jet proximate to and in fluid communication with the second end of the second tube, wherein at least one gas can be supplied through said venturi jet to said port via the second tube, wherein the venturi jet includes a jet having an outlet and a venturi tube, the jet being in fluid communication with a venturi tube and the venturi tube being disposed between the jet and the second end of the second tube, whereby in use the user inspires said at least one gas to ventilate the user and gas expired by the user flows from the port through the second tube and the venturi tube to exit the system, wherein the system additionally includes a second venturi tube having a bore, a first end, a second end in fluid communication with said inlet, and a throat disposed between said first end and said second end, said second venturi tube having a bypass tube which provides fluid communication from a part of the bore between the first end and the throat and a part of the bore at the throat, the bypass tube having a mixer for mixing gas flowing through the bypass tube with a second gas, whereby in use a mixture of gases can be returned to the throat and supplied from the second end of the second venturi tube to the inlet of said first tube.

In a preferred embodiment, the system does not include breath actuated inspiration or expiration valves.

The inventive realisation of the present applicant is that a venturi jet can be used to drive air into a ventilator and can replace valve(s) in the ventilator system.

In accordance with a second aspect of the disclosure, there is provided (but not separately claimed) a method for ventilating a user, comprising providing a system for ventilating a user as defined above, supplying at least one ventilation gas to the inlet of the first tube so that it passes along the first tube, through the outlet and through the port to be inspired by the user in an inspiration phase, receiving expiration gases expired by the user into the port in an expiration phase, wherein at least some of said expiration gases pass through the port, and through the second tube to exit the second tube at its second end, supplying under pressure at least one driving gas through the venturi jet so that said driving gas passes though the second end of the second tube and along the second tube to apply pressure to any expiration gases in the second tube and purge them from the second tube, varying the pressure of the driving gas during the inspiration phase, the expiration phase, and an expiration pause phase between the inspiration phase and the expiration phase so as to control the delivery of ventilation gas to the user.

In accordance with a third aspect of the disclosure, there is provided a venturi tube for use in a medical ventilator, wherein the venturi tube has an inlet at the end of the venturi tube proximate the jet, an outlet at the end or the venturi tube proximate the second end of the second tube, and a throat disposed between the inlet and said outlet, wherein the inlet, the throat and said outlet each define an internal diameter of the venturi tube, and wherein the internal diameter defined by the throat is less than the internal diameter defined by the inlet and said outlet, wherein the internal diameter of the inlet is at least 3 times the internal diameter of the throat, and wherein the internal diameter of the outlet is at least 2 times the internal diameter of the throat.

In a number of preferred embodiments according to the disclosure, the venturi tube may be defined as follows:
- the throat has an internal diameter of 6mm
- the throat has an internal diameter that lies in the range 5mm to 8mm
- the inlet diameter lies in the range 3.5 to 4.5 times the diameter of the throat
- the outlet diameter lies in the range 2.5 to 3.5 times the diameter of the throat
- the throat is closer to the inlet than the outlet.
- the outlet is between 2 and 4.5 times further away from the throat than the inlet
- the throat is a distance of 2.5 to 4 times its own diameter away from the inlet
- the throat is a distance of 6.5 to 12.5 times its own diameter away from the outlet
- the throat is a distance of at least 9 times its own diameter away from the outlet
- the form of the lumen between the inlet and the throat has a trumpet like shape which tapers steeply close to the inlet and where the rate of taper reduces with increasing distance from the inlet.
- the form of the lumen between the inlet and the throat has a trumpet like shape which can be described by a spline fitted to the points between x,y 0,0; 2.5,5; 14,7 and 0,0; 3.5,5; 14,7 where x is the relative position along the axis and y is the relative radius of the lumen at that point.

In accordance with a fourth aspect of the disclosure, there is provided a venturi jet for use in a medical ventilator, including a venturi tube as defined above, a jet having an outlet, the jet being in fluid communication with a venturi tube, wherein the jet and the venturi tube lie on a common axis.

In a number of preferred embodiments according to the disclosure, the venturi jet may be defined as follows:
- the jet is situated within the inlet of the venturi tube
- the internal diameter of the jet is ¼ times the internal diameter of the venturi tube at the throat
- the internal diameter of the jet is between 1/10 and 1/3 times the internal diameter of the venturi tube at the throat
- the jet projects into the venturi tube by 2/3 the internal diameter of the throat
- the jet projects into the venturi tube by between ¼ and 1/6 times the distance from the inlet to the throat

Preferably, the jet and the venturi tube are mechanically linked and those skilled in the art will appreciate that a number of options are available to the designer. In a preferred embodiment, a series of struts or radial spokes are used to connect the jet to the venturi tube. It is necessary that the open area formed between the struts and their attachment points to the jet and venturi tube enclose a greater total area than that of the entrainment orifice to ensure that gas flow into the entrainment orifice is not unnecessarily restricted by the struts.

Variants of the strut arrangement can be imagined including ones in which the position of the jet with respect to the entrainment orifice can be adjusted by, for example, a lead screw which is adjust by a technician or a lead screw that can be adjusted by some form of motor such as a stepper motor or servo motor.

The preferred functions required of a medical ventilator providing pressure control ventilation are as follows:
1. At the onset of inspiration, the pressure applied from the venturi through the breathing circuit to the patient's lungs must rise rapidly to the pressure set by the operator and remain at that pressure until the end of the inspiration period.
   This requires that the venturi achieves and maintains the set pressure while delivering the volume of gas to the patient that is needed to achieve that pressure. Typical volumes (Tidal Volumes or Vt) that may be required lie in the range 100cc to 1000cc and typical pressures (Peak Inspired Pressure or PIP) lie in the range 10cm H2O to 40cm H2O. Some patients, depending upon their age, disease and injury may require values outside these ranges.
2. During expiration, the muscolo-elastic recoil of the patients lungs drives air from the lungs and back through the venturi. This requires that the pressure developed by the venturi is significantly lower during expiration than during inspiration. There is also the requirement that the patient must be able to expel the complete volume of their expired air through the venturi before the next inspiration takes place and with as little resistance to the flow of expired air as possible.

During expiration it is a common clinical requirement to provide a low, constant opposing pressure to the expired gas. The purpose of this expiration 'challenge' pressure is to prevent collapse of alveoli and potentially to encourage the recruitment of larger numbers of alveoli. This pressure is referred to as Positive End Expiratory Pressure or PEEP. Typical values for PEEP lie in the range 5cm H2O to 20cm H2O.

Therefore in a preferred embodiment the venturi design is able to alternate its characteristics between and inspiration state and an expiration state.

In the inspiration state, a second preferred objective of the venturi design is that it achieves an output pressure that can be set by an operator and controlled by the venturi.

In the inspiration state, a third preferred objective of the venturi design is that it achieves the output pressure while delivering whatever inspired volume is required by the patient.

In the expiration state, a fourth preferred objective of the venturi design is that it allows whatever expired volume is required by the patient with as little resistance as possible.

In the expiration state, a fifth preferred objective of the venturi design is that while permitting expiration by the patient, it creates a controllable back pressure that partially opposes the expiration of air by the patient.

The following section describes operation of the venturi. It will refer to 'air' throughout and those skilled in the art will appreciate that the description will apply to another gas or gasses.

In operation, compressed air is passed through the jet, and emerges at high speed through the narrow nozzle. The high speed flow creates a region of low pressure around it, causing ambient air to flow into the entrainment orifice. The entrained air continues to pass through the throat of the venturi, creating a region of slower moving air which is a combination of the volumes of air injected by the nozzle and the entrained ambient air. Along the length of the diffuser, the flow rate slows, increasing the pressure, allowing delivery of a substantial volume of air at the elected pressure to the patient. When the pressure at the distal end of the diffuser is equal to the pressure in the patient's lungs, flow in the diffuser will be disrupted and entrainment of air will be reduced to close to zero. This condition is referred to as 'stalling'. Under this circumstance, no further air will flow into the patient's lungs. However, should the pressure in the patients lungs reduce, then stable flow will re-establish itself in the diffuser, pressure will be developed and flow into the patient's lungs will re-start.

During expiration, the conditions causing entrainment need to be adjusted so that the stalling pressure is substantially reduced. Preferentially, this can be achieved by reducing the pressure of the compressed air applied to the nozzle, but it will be appreciated that other mechanisms, such as changing the position of the jet by moving it closer to or further away from the throat can also provide control of entrainment.

In the limit, if no compressed air flows through the nozzle, then the air pathway from the patients breathing tube through the venturi is equivalent to a short tubular extension with a reduced lumen diameter.

If some compressed air flows from the nozzle during expiration, the expiratory flow will cause the venturi system to stall, unless the expired pressure falls below the pressure generated by the venturi system. In this case, positive end expiratory pressure will have been created, creating the clinical benefits described above.

While the principle of high speed jets of fluid creating a region of low pressure around them is usually referred to as Bernoulli's principle, Bernoulli's equations only consider incompressible fluids and thus more complex analysis with computational fluid dynamics is encouraged to those wishing to explore the impact of design changes to achieve different operating characteristics.

The position of the nozzle with respect to the throat affects the performance of the venturi. Assuming the nozzle is always co-axial with the venturi, placing the nozzle closer to the throat will result in the venturi producing a higher pressure but with a lower volume flow. Conversely, if the nozzle is inserted less far into the venturi so that it is closer to the entrainment orifice, the venturi will produce a greater air flow from having entrained more air, but it will be able to sustain this performance only at a diminished maximum pressure.

The maximum pressure (or stalling pressure of the venturi) can also be increased by reducing the diameter of the throat. It will be apparent that reducing the diameter of the throat will also increase the resistance to airflow from the patient during the expiratory phase.

The length of the diffuser affects the maximum or stall pressure while making little impact on other parameters. Thus lengthening the diffuser increases the maximum pressure that the venturi can produce while a short diffuser significantly limits the performance of the venturi.

A preferred embodiment of the venturi comprises an outlet of the diffuser section which is selected to be a push fit onto standard sized tubing used in breathing circuits.

In this embodiment, the venturi is constructed from a 3D-printable plastic resin but those skilled in the art will appreciate that the design can be easily manufactured by several methods including but not limited to injection moulding, casting and CNC machining.

Materials used may be restricted to include only those that are biocompatible but will include plastics, metals and ceramics, all of which will be familiar to those skilled in the art.

A number of preferred embodiments will now be described with reference to and as illustrated in the accompanying drawings, in which:
Figure 1A is a schematic diagram showing the arrangement of components of a ventilator in accordance with the present invention;
Figure 1B is an alternative representation of the arrangement of Figure 1B with the various components labelled;
Figure 2 is a schematic diagram showing a ventilator system in accordance with the present invention which uses a single venturi;
Figure 3 is a schematic diagram of apparatus for blending oxygen and air for supply to the user;
Figure 4 is a schematic diagram showing a Venturi jet and tube in accordance with the present invention;
Figure 5 a schematic diagram of the Venturi jet and tube of Figure xA showing dimensions; and
Figure 6 to 14 are graphs showing flow rate against time for a number of differently dimensioned Venturi tubes.

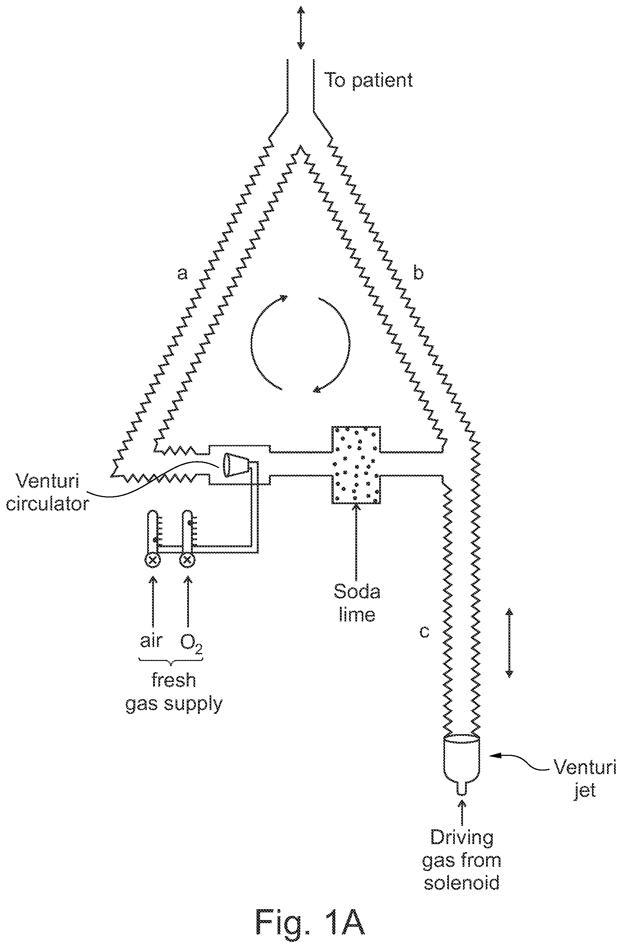

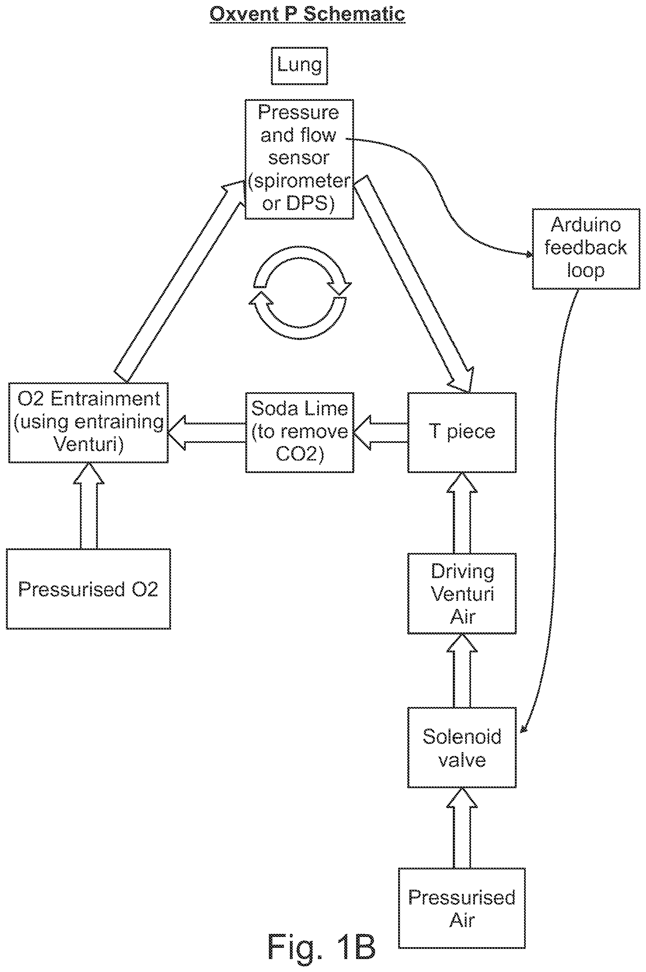

The present invention is a ventilator which is designed to be cheap and scalable while eliminating the silicon valves as far as possible the use of silicon valves. Silicon valves are expensive and sources of failure within conventional ventilators; and their removal is therefore an advantageous design aim. In one embodiment the present invention seeks to achieve this through the use of two venturi jets: one to drive and control flow (through pairing with a solenoid valve), and one to entrain oxygen into the circuit. A schematic diagram showing this set-up is shown in Figures 1A and 1B.

In use, a fresh gas flow (FGF) is driven under pressure via an entraining Venturi to the patient for inhalation. The source of O2 is likely at 4-5 bar in most clinical set ups. The air/O2 mixture formed then moves along inspiration arm (a) to the lungs where oxygen enters the patient.

Crucial to this process is ensuring that O2 concentration at inhalation remains at an acceptable level. This is typically desired at 40% in standard recovering ICU patients, but up to 100% in acutely sick patients. This O2 concentration may rise above the desired level if the entrainment ratio (Total Flow Output/O2 Flow input) is too low at the entraining venturi. This would result in an unsafe amount of oxygen being sent to the patient's lungs. Conversely, the O2 concentration may also fall below the desired level due to either too little O2 being entrained or a CO2 build up in the rebreathing circuit. In conventional ventilators this is avoided through dedicated inspiration/ expiration tubes which are controlled with valves.

The present invention seeks to circumvent this need for valves and separate tubing by ensuring there is a constant flow bias due to the constant stream of O2 in. This results in the CO2 rich air produced on expiration "preferring" to flow back down the non- entraining tube (b) where it exits the system through the driving venturi.

The central connecting tube contains a soda lime canister to purge any CO2 that could potentially pass into the O2 entraining flow and complicate things. Combined, this means that in theory there should be no mechanism for a CO2 build up in the present ventilator so long as a net positive flow towards the lung is observed in the entrainment side. Ideally CO2 concentration should be at 0% in the inspiration flow.

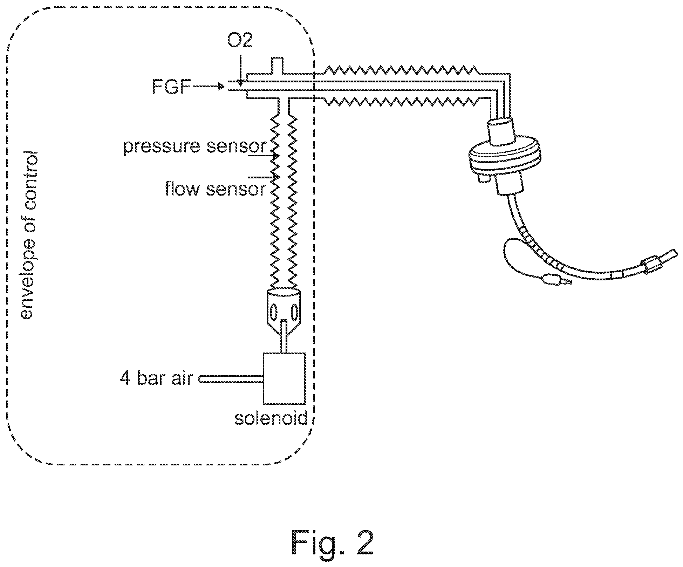

An even simpler embodiment is shown in the system of Figure 2, in which a single venturi jet is employed to (a) allow expired gas to exit the system via the venturi when the expiratory pressure exceeds a specific value and (b) deliver a driving gas to drive fresh gas into the patient's lungs during the inspiration phase. In this embodiment an array of 4 binary solenoids are used to deliver air through the venturi with different outputs (or identical solenoids fitted with different chokes) giving, for example, nominal flows in solenoids A, B, C and D of 2.5, 5, 10 and 20 arbitrary units.

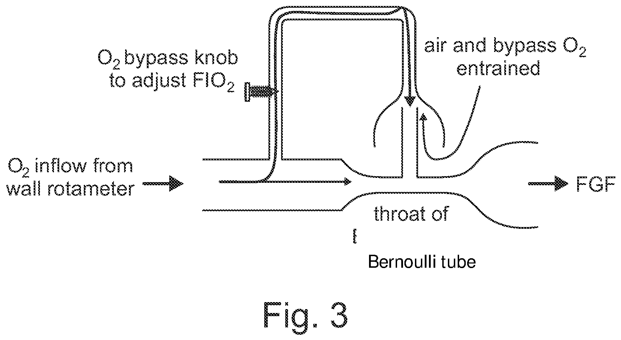

A simple and inexpensive mechanism for blending air and O2 for the fresh gas supply is shown in Figure 3. This is simple to operate and could be printed or moulded very cheaply.

The bypass knob could be calibrated with printed dial markings. Obviously, opening up the bypass channel reduces the flow through the throat, and so reduces the FGF, so there ought to be an adjustment of the O2 rotameter as FIO2 is changed

### Venturi Jet

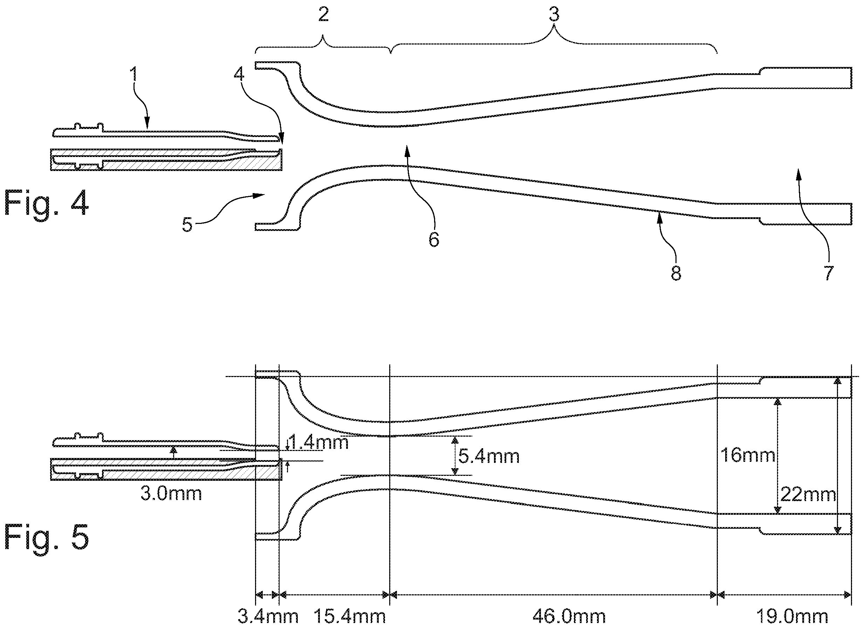

Turning to Figures 4 and 5, the venturi jet comprises two primary components, a jet (1) and a venturi tube (8) which are preferably arranged to lie on a common axis. The jet is characterised in having a tubular body which is narrowed at the end of the jet proximate to the venturi tube to form a nozzle (4).

The venturi tube is a tubular structure having an opening at each end. The opening proximate to the jet is the entrainment orifice and the second opening is the outlet. The lumen of the venturi tube is arranged to reduce in diameter from the entrainment orifice to its narrowest point, the throat (6) before increasing in diameter in the region connecting to the outlet. Preferably, the length of the venturi tube from the entrainment orifice to the throat is shorter than the length from the throat to the outlet.

These devices are widely used in respiratory medicine and anaesthetics to blend air and oxygen, and to deliver high flows of air/O2 to facemasks and CPAP masks. The theory of operation is well known.

A jet of high velocity gas (usually oxygen) exits a needle into surrounding air. Shear forces then drag the stationary air to a higher velocity whilst the oxygen jet is retarded to a lower velocity. At some point along the path, the oxygen and air have a common velocity and are mixed to give an FIO2 determined by the entrainment ratio of the nozzle.

They come with different entrainment ratios, so if the ratio is 3, it will deliver 3 parts air to 1 part O2, giving an O2 concentration of 40% etc. If the O2 flow rate is set at 10L/min, the total O2/air mix will be 40L/min, which should satisfy the patient's peak inspiratory flow

One problem of these devices is that they are sensitive to 'back-pressure'. They work perfectly well delivering a predictable flow with a predictable FIO2 until any back pressure is applied, then the flow drops off. At even modest back-pressures, the flow stalls altogether. The pressure at which this occurs is called the 'stalling pressure'. Stalling pressure can be predicted from modelling, but not surprisingly it depends on the jet size, jet velocity, size of the divergent nozzle outlet.

When inspiratory pressure rises and the flow begins to decelerate, the excess gas from the venturi simply leaks back out of the entrainments ports. Given that the stalling pressure is a function of the driving flow, we can regulate this with a solenoid. We can set the airway pressure at whatever value we like simply by regulating solenoid flow. Importantly also, we can regulate PEEP in exactly the same way. During inspiration we set Pᵢₙₛₚ to whatever value we chose via the solenoid flow, and in expiration we set it to achieve our chosen PEEP value.

When testing the first round of custom venturis we were limited by nozzle diameters that were too large and as such did not entrain enough flow to be a reliable indicator of performance at lower values of entrainment flow. The decision was taken to up the entraining flow until post entrainment flow was reading a baseline of about 25L/min during ventilation. This had the effect of ensuring the speed of flow exiting the nozzle (normally increased by shrinking the nozzle diameter) was increased and entrainment flow would be consistent.

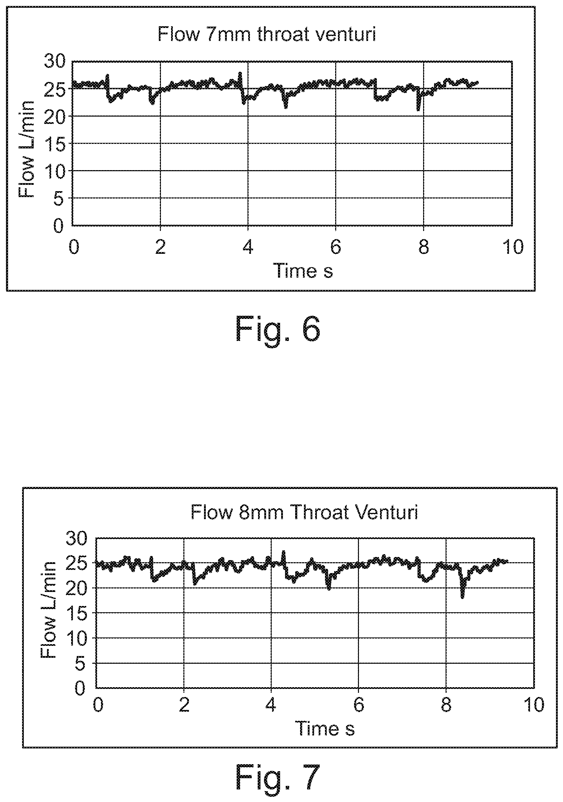

First varying the throat diameter through Figures 6, 7 and 8 we see that decreasing the throat diameter results in a more stable wave form, as well as decreasing the amplitudes of the inspiration and expiration spikes. This is likely due to the flow having less opportunity to reverse on itself resulting in a more consistent direction.

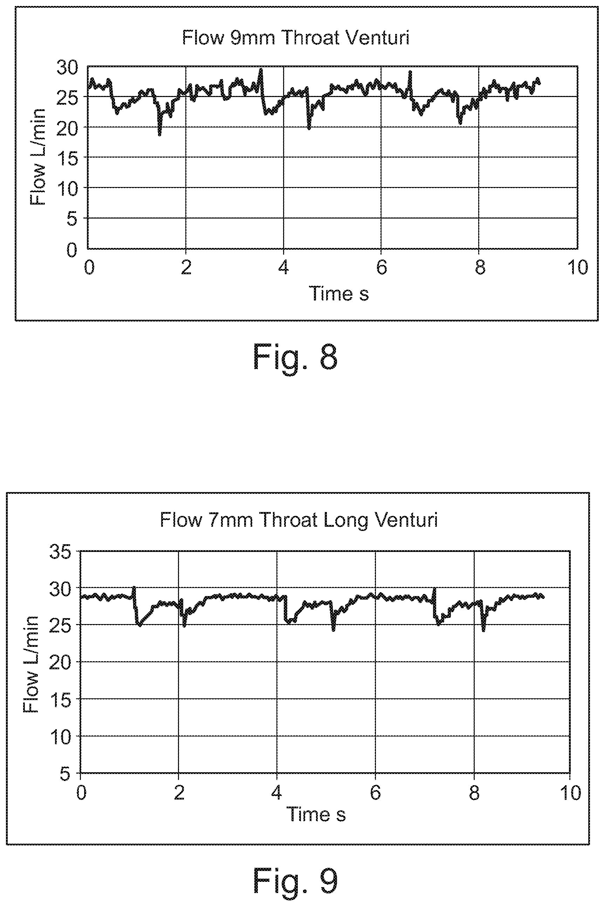

A singular test was also carried out on whether to extend the length of the Venturi. This proved promising as seen in Figure 9, in which we see an even smoother waveform for a 7mm throat than in the shorter 7mm throat venturi (Figure 6). This increase in Venturi length was adopted for all further venturi designs.

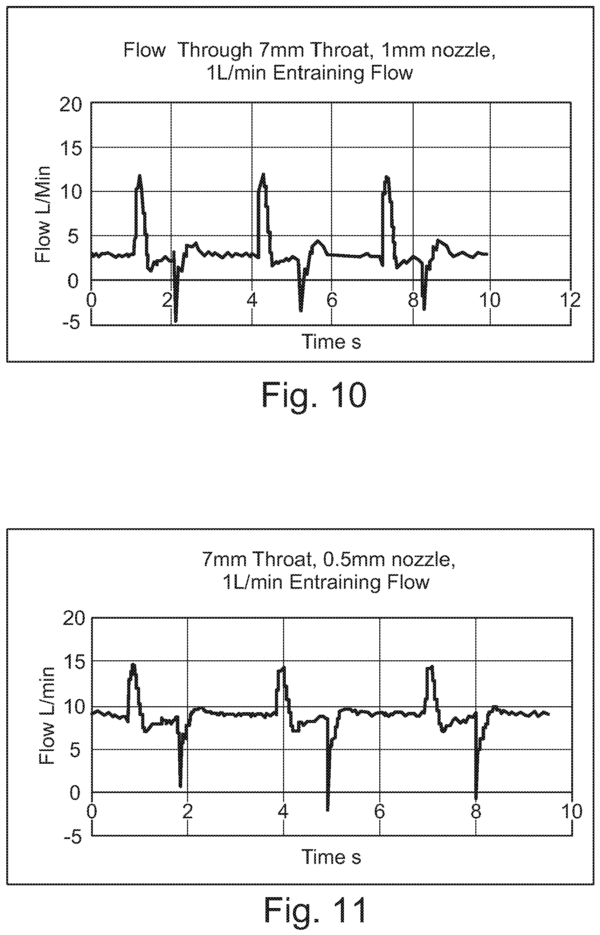

Venturis of varying throat and nozzle diameter were also tested at both 1L/min and 2L/min entraining flow. It was apparent that performance can be quickly improved by decreasing the nozzle diameter. A comparison of Figures 10 and 11 shows that decreasing the nozzle diameter from 1mm to 0.5mm raised the post entrainment flow rate by upwards of 5L/min. This trend was consistent across throat diameters.

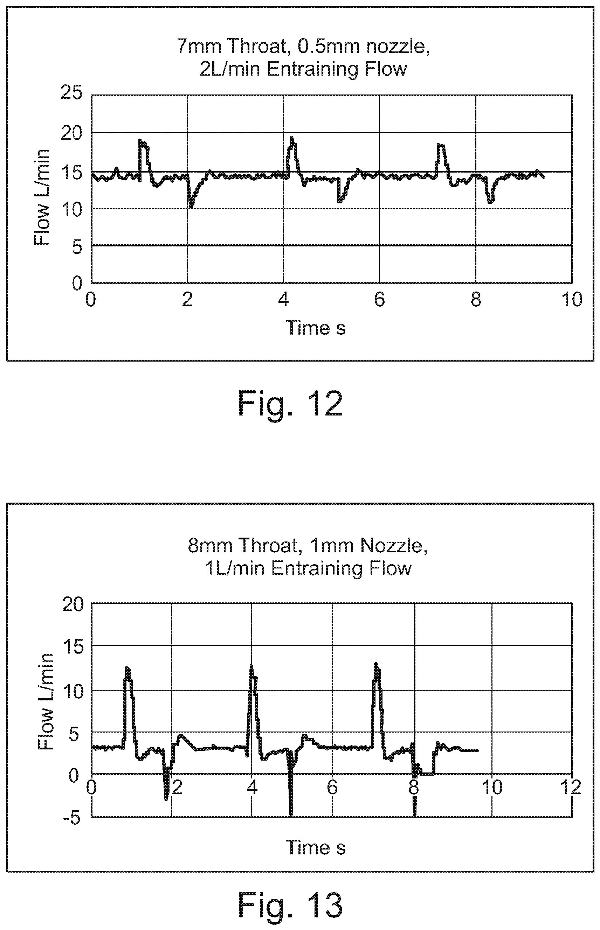

Increasing the entraining flow rate to 2L/min had a dramatic effect on the behaviour of the flow, as the average post entrainment flow rate increased by about 5L/min and the amplitude of the largest troughs also decreased by about 5L/min as shown in Figure 12. It is clear that with even a small increase in O2 flow rate from 1L/min to 2L/min we can expect a very substantial increase in the entrainment performance.

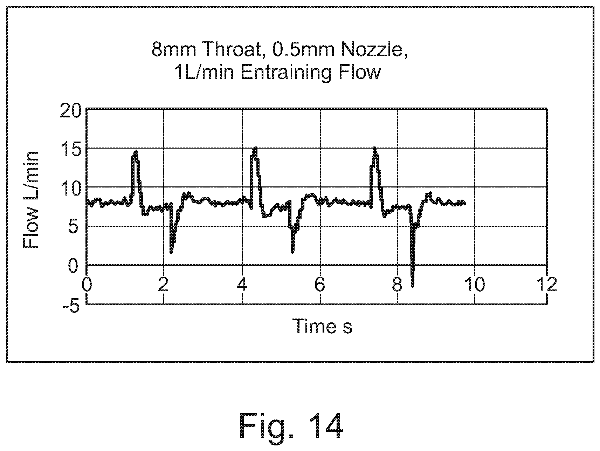

Increasing the throat diameter from 7mm to 8mm did not change a great deal, as shown in Figures 13 and 14.

The amount of flow entrained by the entraining venturi is increased most by decreasing the nozzle diameter and thus, by continuity, increasing the speed of the jet out the other side. This had by far the most impact of any dimension change; however clearly there is a limit to this benefit as the nozzle diameter tends to zero and the boundary layers around the edges of the nozzle play a larger role in slowing the fluid, so a full matrix should be produced to determine the most effective diameter to use to entrain as much flow as possible.

Without wishing to be constrained by theory, it is believed that the following factors apply when designing Venturis for use in the invention:
- Longer diffuser- less circulation- higher stall pressure, better pressure recovery (flow slows down, so get higher pressure return)
- Wider throat - stall pressure decreases - easier to get flow to turn around in throat
- Nozzle further from throat - higher entrainment, lower stall pressure - sucks in more air but easier to stop it

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

## Claims

1. A system for ventilating a user, including
a port for supplying a mixture of gases to the user for inspiration and for receiving a mixture of gases from the user after expiration
a first tube having an inlet for receiving at least one gas to ventilate the user and an outlet in fluid communication with said port for supplying said at least one gas to the port for inspiration by the user,
a second tube having a first end proximate to and in fluid communication with said port and a second end distal to said port,
a venturi jet proximate to and in fluid communication with the second end of the second tube, wherein at least one gas can be supplied through said venturi jet to said port via the second tube, wherein the venturi jet includes a jet having an outlet and a venturi tube, the jet being in fluid communication with a venturi tube and the venturi tube being disposed between the jet and the second end of the second tube,
whereby in use the user inspires said at least one gas to ventilate the user and gas expired by the user flows from the port through the second tube and the venturi tube to exit the system
**characterized in that** the system additionally includes
a second venturi tube having a bore, a first end, a second end in fluid communication with said inlet, and a throat disposed between said first end and said second end,
said second venturi tube having a bypass tube which provides fluid communication from a part of the bore between the first end and the throat and a part of the bore at the throat,
the bypass tube having a mixer for mixing gas flowing through the bypass tube with a second gas,
whereby in use a mixture of gases can be returned to the throat and supplied from the second end of the second venturi tube to the inlet of said first tube.

2. A system as claimed in claim 1, additionally including a valve for controlling the proportion of gas passing through the second venturi tube which is diverted down the bypass tube.

3. A system as claimed in any preceding claim, wherein at least a section of the first tube is coaxial with and located within at least a section of the second tube.

4. A system as claimed in any preceding claim, wherein the jet and the venturi tube lie on a common axis.

5. A system as claimed in any preceding claim, wherein the jet and the venturi tube are mechanically linked.

6. A system as claimed in any preceding claim, wherein the venturi tube has an inlet at the end of the venturi tube proximate the jet, an outlet at the end of the venturi tube proximate the second end of the second tube, and a throat disposed between the inlet and said outlet, wherein the inlet, the throat and said outlet each define an internal diameter of the venturi tube, and wherein the internal diameter defined by the throat is less than the internal diameter defined by the inlet and said outlet.

7. A system as claimed in claim 6, wherein the internal diameter of the inlet is at least 3 times the internal diameter of the throat, and wherein the internal diameter of the outlet is at least 2 times the internal diameter of the throat.

8. A system as claimed in claim 6 or 7, wherein the length of the venturi tube from the inlet to the throat is shorter than the length of the venturi tube from the throat to said outlet.

9. A system as claimed in any preceding claim, wherein the position of the jet relative to the venturi tube is adjustable.

10. A system as claimed in any preceding claim, wherein the first tube includes therein a substance which reacts with carbon dioxide to remove carbon dioxide expired by the user from the first tube.

11. A system as claimed in any preceding claim, wherein the venturi jet has an entrainment ratio of about 3.

12. A system as claimed in any preceding claim which does not include any mechanical valves in the first tube, the second tube or the venturi jet.

13. A system as claimed in any preceding claim additionally including a source of at least one gas for supplying said at least one gas to the inlet of the first tube.

14. A system as claimed in any preceding claim, additionally including a third tube which provides a fluid communication between the second tube and the inlet of the first tube, in order that gas expired by the user may be recirculated from the second tube into the first tube.

15. A system as claimed in claim 14, wherein the third tube includes therein a substance which reacts with carbon dioxide to remove carbon dioxide expired by the user from the third tube.

## Patentansprüche

1. Ein System zur Beatmung eines Anwenders, umfassend
einen Anschluss zur Zufuhr eines Gasgemischs an den Anwender zum Einatmen und zur Aufnahme eines Gasgemischs vom Anwender nach dem Ausatmen
ein erstes Rohr mit einem Einlass zum Aufnehmen mindestens eines Gases zur Beatmung des Anwenders und einem Auslass, der in Fluidverbindung mit dem Anschluss steht, um das mindestens eine Gas dem Anschluss zur Einatmung durch den Anwender zuzuführen,
ein zweites Rohr mit einem ersten Ende in der Nähe des Anschlusses und in Fluidverbindung mit diesem sowie einem zweiten Ende, das vom Anschluss entfernt ist,
eine Venturi-Düse, die sich in der Nähe des zweiten Endes des zweiten Rohrs befindet und mit diesem in Fluidverbindung steht, wobei mindestens ein Gas durch die Venturi-Düse über das zweite Rohr dem Anschluss zugeführt werden kann, wobei die Venturi-Düse ein Düsenelement mit einem Auslass und ein Venturi-Rohr umfasst, wobei das Düsenelement mit einem Venturi-Rohr in Fluidverbindung steht und das Venturi-Rohr zwischen dem Düsenelement und dem zweiten Ende des zweiten Rohrs angeordnet ist,
wodurch der Anwender im Betrieb das mindestens eine Gas einatmet, um den Anwender zu beatmen, und vom Anwender ausgeatmetes Gas von dem Anschluss durch das zweite Rohr und das Venturi-Rohr strömt, um das System zu verlassen
, **dadurch gekennzeichnet, dass** das System zusätzlich umfasst
ein zweites Venturi-Rohr mit einer Bohrung, einem ersten Ende, einem zweiten Ende, das in Fluidverbindung mit dem Einlass steht, und einer zwischen dem ersten Ende und dem zweiten Ende angeordneten Verengung,
wobei das zweite Venturi-Rohr ein Bypass-Rohr aufweist, das eine Fluidverbindung zwischen einem Teil der Bohrung zwischen dem ersten Ende und der Verengung und einem Teil der Bohrung an der Verengung herstellt,
wobei das Bypass-Rohr einen Mischer aufweist, um das durch das Bypass-Rohr strömende Gas mit einem zweiten Gas zu mischen,
wodurch im Betrieb ein Gasgemisch in die Verengung zurückgeführt und vom zweiten Ende des zweiten Venturi-Rohrs zum Einlass des ersten Rohrs geleitet werden kann.

2. Ein System nach Anspruch 1, das zusätzlich ein Ventil zur Steuerung des Anteils des durch das zweite Venturi-Rohr strömenden Gases umfasst, das in das Bypass-Rohr umgeleitet wird.

3. System nach einem der vorstehenden Ansprüche, wobei zumindest ein Abschnitt des ersten Rohrs koaxial zu zumindest einem Abschnitt des zweiten Rohrs ist und sich innerhalb desselben befindet.

4. System nach einem der vorstehenden Ansprüche, wobei das Düsenelement und das Venturi-Rohr auf einer gemeinsamen Achse liegen.

5. Ein System nach einem der vorstehenden Ansprüche, wobei das Düsenelement und das Venturi-Rohr mechanisch miteinander verbunden sind.

6. Ein System nach einem der vorstehenden Ansprüche, wobei das Venturi-Rohr einen Einlass am Ende des Venturi-Rohrs in der Nähe des Düsenelements, einen Auslass am Ende des Venturi-Rohrs in der Nähe des zweiten Endes des zweiten Rohrs und eine zwischen dem Einlass und dem Auslass angeordnete Verengung aufweist, wobei der Einlass, die Verengung und der Auslass jeweils einen Innendurchmesser des Venturi-Rohrs definieren und wobei der durch die Verengung definierte Innendurchmesser kleiner ist als der durch den Einlass und den Auslass definierte Innendurchmesser.

7. System nach Anspruch 6, wobei der Innendurchmesser des Einlasses mindestens das Dreifache des Innendurchmessers der Verengung beträgt und wobei der Innendurchmesser des Auslasses mindestens das Zweifache des Innendurchmessers der Verengung beträgt.

8. System nach Anspruch 6 oder 7, wobei die Länge des Venturi-Rohrs vom Einlass bis zur Verengung kürzer ist als die Länge des Venturi-Rohrs von der Verengung bis zum Auslass.

9. System nach einem der vorstehenden Ansprüche, wobei die Position des Düsenelements relativ zum Venturi-Rohr einstellbar ist.

10. Ein System nach einem der vorstehenden Ansprüche, wobei das erste Rohr eine Substanz enthält, die mit Kohlenstoffdioxid reagiert, um vom Anwender ausgeatmetes Kohlenstoffdioxid aus dem ersten Rohr zu entfernen.

11. System nach einem der vorstehenden Ansprüche, wobei die Venturi-Düse ein Mitreißverhältnis von etwa 3 aufweist.

12. System nach einem der vorstehenden Ansprüche, das keine mechanischen Ventile im ersten Rohr, im zweiten Rohr oder in der Venturi-Düse enthält.

13. System nach einem der vorstehenden Ansprüche, das zusätzlich eine Quelle für mindestens ein Gas umfasst, um das mindestens eine Gas dem Einlass des ersten Rohrs zuzuführen.

14. Ein System nach einem der vorstehenden Ansprüche, das zusätzlich ein drittes Rohr umfasst, das eine Fluidverbindung zwischen dem zweiten Rohr und dem Einlass des ersten Rohrs herstellt, damit vom Anwender ausgeatmetes Gas aus dem zweiten Rohr in das erste Rohr rezirkuliert werden kann.

15. System nach Anspruch 14, wobei das dritte Rohr eine Substanz enthält, die mit Kohlenstoffdioxid reagiert, um vom Anwender ausgeatmetes Kohlenstoffdioxid aus dem dritten Rohr zu entfernen.

## Revendications

1. - Système de ventilation d'un utilisateur, comprenant
un orifice pour fournir un mélange de gaz à l'utilisateur pour inhalation et pour recevoir un mélange de gaz provenant de l'utilisateur après expiration ;
un premier tube ayant une entrée pour recevoir au moins un gaz pour ventiler l'utilisateur et une sortie en communication fluidique avec ledit orifice pour fournir ledit au moins un gaz à l'orifice pour son inhalation par l'utilisateur ;
un deuxième tube comportant une première extrémité à proximité de et en communication fluidique avec ledit orifice, et une seconde extrémité distale par rapport audit orifice ;
un jet de Venturi à proximité de et en communication fluidique avec la seconde extrémité du deuxième tube, au moins un gaz pouvant être fourni à travers ledit jet de Venturi audit orifice par l'intermédiaire du deuxième tube, le jet de Venturi comprenant un jet ayant une sortie et un tube de Venturi, le jet étant en communication fluidique avec un tube de Venturi et le tube de Venturi étant disposé entre le jet et la seconde extrémité du deuxième tube,
ce par quoi, lors de l'utilisation, l'utilisateur inhale ledit au moins un gaz pour ventiler l'utilisateur et le gaz expiré par l'utilisateur s'écoule à partir de l'orifice à travers le deuxième tube et le tube de Venturi pour sortir du système,
**caractérisé par le fait que** le système comprend en outre
un second tube de Venturi ayant un alésage, une première extrémité, une seconde extrémité en communication fluidique avec ladite entrée, et un étranglement disposé entre ladite première extrémité et ladite seconde extrémité,
ledit second tube de Venturi ayant un tube de dérivation qui permet une communication fluidique entre une partie de l'alésage entre la première extrémité et l'étranglement et une partie de l'alésage à l'étranglement,
le tube de dérivation ayant un mélangeur pour mélanger le gaz s'écoulant à travers le tube de dérivation avec un second gaz,
ce par quoi, lors de l'utilisation, un mélange de gaz peut être renvoyé à l'étranglement et fourni de la seconde extrémité du second tube de Venturi à l'entrée dudit premier tube.

2. - Système selon la revendication 1, comprenant en outre une soupape pour contrôler la proportion de gaz passant à travers le second tube de Venturi qui est déviée vers le tube de dérivation.

3. - Système selon l'une quelconque des revendications précédentes, dans lequel au moins une section du premier tube est coaxiale à et située à l'intérieur d'au moins une section du deuxième tube.

4. - Système selon l'une quelconque des revendications précédentes, dans lequel le jet et le tube de Venturi s'étendent sur un axe commun.

5. - Système selon l'une quelconque des revendications précédentes, dans lequel le jet et le tube de Venturi sont liés mécaniquement.

6. - Système selon l'une quelconque des revendications précédentes, dans lequel le tube de Venturi a une entrée à l'extrémité du tube de Venturi à proximité du jet, une sortie à l'extrémité du tube de Venturi à proximité de la seconde extrémité du deuxième tube, et un étranglement disposé entre l'entrée et ladite sortie, l'entrée, l'étranglement et ladite sortie définissant chacun un diamètre interne du tube de Venturi, et le diamètre interne défini par l'étranglement étant inférieur au diamètre interne défini par l'entrée et ladite sortie.

7. - Système selon la revendication 6, dans lequel le diamètre interne de l'entrée est d'au moins 3 fois le diamètre interne de l'étranglement, et le diamètre interne de la sortie est d'au moins 2 fois le diamètre interne de l'étranglement.

8. - Système selon la revendication 6 ou 7, dans lequel la longueur du tube de Venturi de l'entrée à l'étranglement est inférieure à la longueur du tube de Venturi entre l'étranglement et ladite sortie.

9. - Système selon l'une quelconque des revendications précédentes, dans lequel la position du jet par rapport au tube de Venturi est réglable.

10. - Système selon l'une quelconque des revendications précédentes, dans lequel le premier tube comprend intérieurement une substance qui réagit avec le dioxyde de carbone pour retirer le dioxyde de carbone expiré par l'utilisateur à partir du premier tube.

11. - Système selon l'une quelconque des revendications précédentes, dans lequel le jet de Venturi a un rapport d'entraînement d'environ 3.

12. - Système selon l'une quelconque des revendications précédentes, qui ne comporte pas de soupapes mécaniques dans le premier tube, le deuxième tube ou le jet de Venturi.

13. - Système selon l'une quelconque des revendications précédentes, comprenant en outre une source d'au moins un gaz pour fournir ledit au moins un gaz à l'entrée du premier tube.

14. - Système selon l'une quelconque des revendications précédentes, comprenant en outre un troisième tube qui assure une communication fluidique entre le deuxième tube et l'entrée du premier tube, afin que le gaz expiré par l'utilisateur puisse être recyclé du deuxième tube dans le premier tube.

15. - Système selon la revendication 14, dans lequel le troisième tube comprend intérieurement une substance qui réagit avec le dioxyde de carbone pour retirer le dioxyde de carbone expiré par l'utilisateur à partir du troisième tube.
